(19) Europäisches Patentamt

European Patent Office

Office européen des brevets

(11) **EP 1 142 871 A1**

(12) **EUROPEAN PATENT APPLICATION**
published in accordance with Art. 158(3) EPC

(43) Date of publication:
**10.10.2001 Bulletin 2001/41**

(51) Int Cl.[7]: **C07D 201/04**, B01J 29/40
// C07B61:00

(21) Application number: **99961387.0**

(22) Date of filing: **24.12.1999**

(86) International application number:
**PCT/JP99/07301**

(87) International publication number:
**WO 00/39084 (06.07.2000 Gazette 2000/27)**

(84) Designated Contracting States:
**AT BE CH CY DE DK ES FI FR GB GR IE IT LI LU MC NL PT SE**

(30) Priority: **25.12.1998 JP 36938098**

(71) Applicant: **MITSUBISHI CHEMICAL CORPORATION**
**Chiyoda-ku, Tokyo 100-0005 (JP)**

(72) Inventors:
• **FUJITA, Naoko, Mitsubishi Chemical Corp.**
  **Yokohama-shi, Kanagawa 227-8502 (JP)**

• **MAL, Nawalkishor, Mitsubishi Chemical Corp.**
  **Yokohama-shi, Kanagawa 227-8502 (JP)**
• **SETOYAMA, Tohru, Mitsubishi Chemical Corp.**
  **Yokohama-shi, Kanagawa 227-8502 (JP)**

(74) Representative: **HOFFMANN - EITLE**
**Patent- und Rechtsanwälte**
**Arabellastrasse 4**
**81925 München (DE)**

(54) **OXIDE CATALYSTS AND PROCESS FOR PRODUCING $g(e)-CAPROLACTAM BY USING THE SAME**

(57) In a method for producing ε-caprolactam by reacting cyclohexanone with a nitrogen source and oxygen, the reaction is carried out in the presence of an oxide catalyst containing one or more elements M(s) selected from the elements belonging to the groups 3 to 12 in the periodic table, thereby producing ε-caprolactam in one stage and at a high selectivity coefficient.

FIG. 1

EP 1 142 871 A1

**Description**

TECHNICAL FIELD

The present invention relates to a method for directly producing ε-caprolactam from cyclohexanone, nitrogen source and oxygen and to oxide catalysts used therefor. ε-caprolactam is a compound that is important in industry as a starting material for nylon-6.

BACKGROUND ART

Several methods have been known for producing ε-caprolactam. The method that is most widely employed on an industrial scale is a method in which cyclohexanone is reacted with hydroxylamine to form cyclohexanone oxime, which is then subjected to Beckmann rearrangement by sulfuric acid to produce ε-caprolactam. Hydroxylamine is produced by oxidizing ammonia to produce nitrogen monoxide (NO) and then reducing it. This method has the problems that the process is long and that it requires sulfuric acid in the step of producing hydroxylamine and in the step of Beckmann rearrangement, which sulfuric acid must finally be neutralized with ammonia into ammonium sulfate so that a large amount of ammonium sulfate is discharged.

Accordingly, many efforts have hitherto been made with view to developing a new method for producing ε-caprolactam. For example, Japanese Patent Application Laid-open Nos. Sho 62-59256 and Sho 63-130575 disclose reacting cyclohexanone, ammonia and hydrogen peroxide in a liquid phase in the presence of zeolite TS-1 of MFI type structure containing titanium to produce cyclohexanone oxime.

Also, U. S. Patent 4,163,756, J. Cat., 70 (1981), p.72-83, p.84-91, J. Cat., 72 (1981), p.66-74, J. Amer. Chem. Soc., 102 (1980), p.1453, and Catalysis Letters, 11 (1991), p.285-294 disclose contacting gas containing cyclohexane, ammonia and oxygen with silica or catalyst composed mainly of silica to produce cyclohexanone oxime.

Japanese Patent Application Laid-open Nos. Sho 62-123167 and Sho 63-54358 disclose producing ε-caprolactam by Beckmann rearrangement of cyclohexanone oxime in a gas phase using MFI type zeolite containing substantially no aluminum as the catalyst. In this method, the reaction is performed at a reaction temperature as high as 350°C.

On the other hand, with regard to the method for producing ε-caprolactam from cyclohexanone, a method for producing ε-caprolactam by use of an alumina-silica catalyst in reacting cyclohexanone with ammonia and an oxidation agent in a two-stage reactor is disclosed in J. N. Armor, J. Catal. 70 (1981), p.72 cited above. U. S. Patent 4,163,756 discloses a method for producing ε-caprolactam from cyclohexanone using two types of catalyst, i.e., γ-alumina and La containing molecular sieve in two reaction regions. Also, R. Prasad et al., J. Cat. 161 (1996), p.373 discloses a method for producing ε-caprolactam by reacting cyclohexanone with ammonia and nitrogen oxide in a liquid phase using an alumina-silica catalyst.

DISCLOSURE OF THE INVENTION

Although some improvements have been proposed on the method for producing ε-caprolactam as described above, most of the conventional methods relate to a multi-stage process through the stages : cyclohexanone, cyclohexanone oxime and ε-caprolactam, which form subsequently in that order. There are problems that costs such as construction cost and variable cost are to be incurred. Also, as described above, Prasad et al., J. Cat. 161 (1996), p.373 discloses the method for producing ε-caprolactam from cyclohexanone in a single reactor in one stage. However, the reexamination performed by the present inventors demonstrated that almost no ε-caprolactam was produced and was not reproducible.

In view of the above problems, the present inventors have made extensive research and as a result they have found that use of specified oxide catalysts enables production of ε-caprolactam from cyclohexanone in one step at a high selectivity with preventing a decrease in the activity of the catalysts, thereby completing the present invention.

That is, a first aspect of the present invention relates to a method for producing ε-caprolactam comprising allowing cyclohexanone to react with a nitrogen source and oxygen in the presence of an oxide catalyst containing one or more elements M(s) selected from belonging to the groups 3 to 12 in the periodic table.

Further, a second aspect of the present invention relates to an oxide catalyst for producing ε-caprolactam having the following characteristics 1) to 3):

1) containing one or more elements M(s) selected from the elements belonging to the groups 3 to 12 in the periodic table;

2) having an ammonia desorption amount of 0.5 mmol-$NH_3$/g-cat or less in temperature programmed desorption analysis of ammonia; and

3) having an aluminum content of 1 wt% or less.

Hereinafter, the present invention will be described in detail.

The catalyst used in the present invention is an oxide catalyst that contains one or more elements M(s) selected from the elements belonging to the groups 3 to 12 in the periodic table and optionally silicon as needed. Among the one or more elements M(s) selected from the elements belonging to the groups 3 to 12 in the periodic table, it is preferred that the catalyst contain at least one element selected from the elements belonging to the groups 4 to 8 in the periodic table. If the one or more elements M(s) selected from the elements belonging to the groups 3 to 12 in the periodic table include a plurality of elements, it is preferred that each of them is an element selected from the elements belonging to the groups 4 to 8. Furthermore, the element selected from the elements belonging to the groups 4 to 8 is desirably at least one selected from titanium (Ti), niobium (Nb), tantalum (Ta), hafnium (Hf), zirconium (Zr) and tungsten (W), preferably titanium, niobium, zirconium or tungsten, and particularly titanium is preferred in view of high selectivity of $\varepsilon$-caprolactam produced by the reaction catalyzed by the oxide catalyst containing such element or elements.

The oxide catalyst may be amorphous or crystalline. Crystalline oxides are preferred. Composite oxides, particularly zeolite is preferred. The elements M(s) selected from the elements belonging to the groups 3 to 12 may be present within the lattice or outside the lattice in the case of crystalline oxides. The "crystalline oxides" as used herein refers to oxides composed of crystalline substances. The "composite oxides" as used herein refers to oxides in which two or more metal components coexist. The crystalline oxides as used herein don't refer to mesoporous materials.

The oxide catalyst of the present invention is preferably a catalyst having a small acididy, for example a catalyst that has an acidity equivalent to or less than that of alumina (JRC-AL0-4), a reference catalyst of Catalysis Society of Japan. The acid amount can be evaluated, for example, by temperature programmed desorption (TPD) analysis of ammonia. From the viewpoint of acid intensity, a weak catalyst is preferred. The evaluation of acid intensity may also be performed by temperature programmed desorption analysis of ammonia. More conveniently, acid intensity can be measured using Hammett's indicator.

The oxide catalyst of the present invention has an ammonia desorption amount in temperature programmed desorption (TPD) analysis of ammonia is preferably equivalent to or less than that of alumina (JRC-AL0-4). More specifically, preferred is a catalyst having an acid amount of 0.5 mmol-$NH_3$/g-cat or less, more preferably 0.3 mmol-$NH_3$/g-cat or less, and most preferably 0.1 mmol-$NH_3$/g-cat. The "mmol-$NH_3$/g-cat" represents an ammonia desorption amount (mmol) per g of an oxide catalyst. With regard to acid intensity in terms of acid intensity function Ho as measured with Hammett's indicator, the oxide catalyst of the present invention is a catalyst having an acid intensity function Ho of usually greater than -3.0, preferably greater than +1.5 and more preferably greater than +3.3, particularly preferably greater than +4.0.

The ammonia desorption amount in temperature programmed desorption analysis described above is measured by letting the catalyst to absorb ammonia to saturation at a predetermined temperature, removing the ammonia that weakly binds to the catalyst and ammonia in the atmosphere by deaeration or the like, elevating the temperature to have ammonia desorbed, and determining the amount of the desorbed ammonia. This value is measured typically under the following conditions in the present invention.

<Measuring apparatus>

Measuring apparatus: Automatic Temperature Programmed Desorption Analyzer (TP-5000) produced by Ookura Riken Co., Ltd.

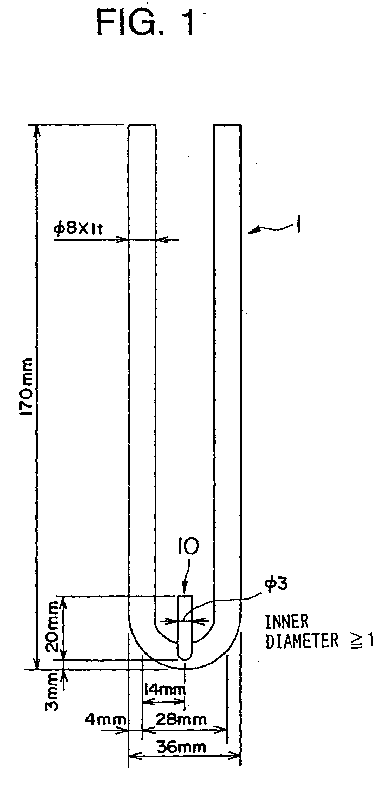

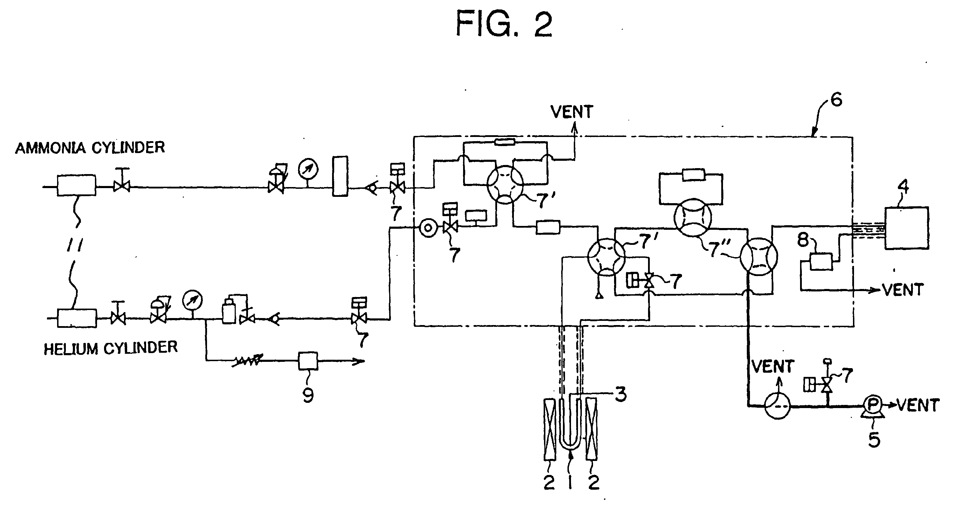

The outline of the above apparatus is described in "Specification of Automatic Temperature Programmed Desorption analyzer (TP-5000)" written by Ookura Riken Co., Ltd. The outline of the apparatus is shown in Fig. 1. Fig. 2 illustrates the reaction tube used. In Fig. 2, 1 denotes a reaction tube, 2 denotes an electric furnace (whose temperature is controlled by a programmed temperature controller), 3 denotes a temperature indicator, 4 denotes a mass spectrometer, 5 denotes an oil-sealed rotary vacuum pump, 6 denotes a constant temperature oven, 7 denotes an electromagnetic valve, 7' denotes a 6-way electromagnetic valve, 7" denotes a 4-way electromagnetic valve, 8 denotes a thermal conductivity detector (sample side), 9 denotes a thermal conductivity detector (reference side), 10 denotes a thermocouple slot, and 11 denotes a pressure reducing valve.

<Measuring conditions>

Carrier gas (helium): Helium gas produced by Union Helium Co.

Ammonia: Liquid ammonia (purity: 99.9% or more) produced by Showa Denko K.K.
(The carrier gas and ammonia were used as they were without performing purification or the like.)
Amount of the catalyst used for the measurement: 200 mg The reaction tube used for the measurement: The reaction tube shown in Fig. 2 was used.

The measurement was performed according to the automatic control/metering program for TP-5000.

<Procedures of measurement>

(1) Pretreatment
The temperature was elevated to 470°C (internal temperature: 500°C) in 45 minutes at a pressure of 1 torr or less and kept at 470°C for 30 minutes.
(2) $NH_3$ pulse adsorption

Set at 100°C (internal temperature: 110°C)
Time for terminating 1 pulse: 3 minutes
Volume of $NH_3$ pulse (measuring tube): 0.953 cc (converted to 0°C, 1 atm.)
Pressure of carrier gas (helium): 0.05 MPa

(Set conditions for $NH_3$ pulse)

Pulse terminating condition: The amount of non-adsorbed $NH_3$ in three pulses from the final pulse falls in an error range of ±3%.
Maximum pulse time: 10 times
When the pulse terminating condition is not reached after pulse was input in several times, the pulse input is terminated when this number of times is reached.
Minimum pulse time: 4 times
Pulses are input at least this number of times regardless of the pulse terminating condition.
Maximum He purging time after $NH_3$ pulse (waiting time for stabilizing the TCD): 2 minutes
Time for introducing pulse gas into piping: 30 seconds
Time for adjusting the inside of the measuring tube to atmosphere pressure: 20 seconds
Time for detecting 1 peak of the palse: 360 seconds

(3) Purge, deaeration treatment (vacuum deaeration, 100°C, 30 minutes)
(4) Confirmation of stabilization of TCD (thermal conductivity detector) (100°C)
(5) Programmed temperature desorption of $NH_3$
The temperature was elevated from 100°C to 800°C at a rate of 10°C/min and kept at 800°C for 20 minutes
Carrier gas flow rate: 30 ml/min
(6) Standard pulse measurement ($NH_3$ pulse fed not through a reaction tube packed with a catalyst is called a standard pulse (volume of $NH_3$ pulse: 0.953 cc; 0°C, 1 atm), the area of which is measured and ammonia desorption amounts are converted based on this area).
(7) Other conditions

Detector: TCD detector (set current 3) and mass spectrometer
Line piping (the dotted line in Fig. 2) and constant temperature oven were kept at 100°C.
(The piping was all 1/8 inch except for the purged portion (thick line in fig. 2: 1/4 inch).)
On the upper surface of the reaction tube was attached by a heat insulator.
Electric furnace control: Auto-tuning was already practiced.

After the $NH_3$ pulse and before the $NH_3$ programmed temperature desorption(5) was started, the time required for purge, deaeration treatment ((3)) and confirmation of stabilization of TCD ((4)) was about 43 minutes. The obtained data was analyzed with waveform editing/analyzing program analysis software (Type MPS-880 waveform editing program) produced by Ookura Riken Co., Ltd. to effect waveform separation of each peak and calculation of areas, and ammonia desorption amounts were obtained from the ratio of the area of the standard pulse of the thermal conductivity detector (TCD) to the area of desorbed ammonia. Note that alumina (JRC-AL0-4), reference catalyst of the Japan Catalyst Society when measured under the same conditions as above showed an ammonia desorption amount of 0.11 to 0.36 mmol-$NH_3$/g-cat.

The oxide catalyst of the present invention preferably has an aluminum content of 1 wt% or less, more preferably 0.5 wt% or less, particularly preferably 0.1 wt% or less, and most preferably 0.05 wt% or less in view of improved selectivity of ε-caprolactam produced.

The concentration of the one or more elements M(s) - selected from the elements belonging to the groups 3 to 12 in the periodic table is usually 0.01 mol% or more, preferably 0.2 mol% or more and most preferably 0.51 mol% or more.

When the oxide catalyst of the present invention contains silicon, the atomic ratio of silicon to the element M (Si/M) in the oxide catalyst is preferably 20 or more, more preferably 30 or more, and particularly preferably 50 or more. If the atomic ratio is too low, generally the selectivity of ε-caprolactam and by-produced cyclohexanone oxime is decreased. On the other hand, if the atomic ratio is too large, the element M exhibits substantially no catalytic action. Therefore, the atomic ratio (Si/M) is usually 10 to 200, preferably 20 to 190, and particularly preferably 50 to 150.

The oxide catalyst of the present invention acts as a catalyst when used singly. However, plural kinds of oxide catalyst may be used in admixture.

As for the synthesis method, the oxide catalyst of the present invention where crystalline composite oxide containing both one or more elements M(s) selected from the elements belonging to the groups 3 to 12 and silicon can be produced, for example, from a compound of the element M and a silicon compound by a known method for synthesizing zeolite. For example, there can be used those obtained by the method in which under coexistence of tetrapropylammonium salt as a structure-directing agent, a silica material and a material of the element M are subjected to hydrothermal synthesis treatment in the presence of an alkaline material. Also, there can be used those obtained by the method in which MFI type zeolite containing aluminum is subjected to acid treatment or the like to remove aluminum and the element M is introduced to the site where aluminum has been desorbed.

The starting material of the components of the one or more elements M(s) selected from the elements belonging to the groups 3 to 12 in the periodic table is not particularly limited but the salts of these metals, for example, nitrates, sulfurates, acetates, oxalates, carbonates, halides such as chlorides and bromides, and metal alkoxides may be used. Among them, metal alkoxides, nitrates, acetates, and oxalates are preferred.

When preparing composite oxides containing silicon, the starting material for silicon is not particularly limited but commercially available silica, tetraalkyl orthosilicate, aerosil, colloidal silica, sodium silicate and the like are exemplified.

Also, there can be used silica-supported catalyst prepared, for example, by impregnating a preliminarily formed silica carrier with a solution containing one or more compounds of the one or more elements M(s) selected from the elements belonging to the groups 3 to 12 in the periodic table, and converting the impregnated compound or compounds of the element or elements into oxide or oxides by thermal decomposition, hydrolysis followed by thermal decomposition or the like.

The atmosphere in which the thermal decomposition is performed for preparing the catalyst of the present invention may contain oxygen or contain no oxygen. In the atmosphere containing oxygen, substances that can be oxidized, such as organic substances, if any, are oxidized or burned to cause local heat generation, which may inhibit the reactivity of the resulting catalyst. Accordingly, it is desirable to sufficiently control heat generation. The heat decomposition may be carried out at 400°C or more. However, condensation reaction of cyclohexanone or the like that takes place as a side reaction can also occur due to the acid substance of the carrier so that it is preferred to practice the treatment at a temperature of 500°C or more.

In the production method of ε-caprolactam according to the present invention, cyclohexanone is reacted with a nitrogen source and oxygen in the presence of the oxide catalyst.

The reaction may be carried out either in a liquid phase or in a gas phase. Usually, it is performed in a gas phase. The nitrogen source includes ammonia and any ammonia generating source, i.e., any substance that generates ammonia in the system, such as ammonia water, ammonium salts, and urea. In particular, it is preferred to use ammonia or urea. In the gas supplied to the reaction, cyclohexanone, ammonia and oxygen may be usually present in any molar ratios selected from the ranges of 0.001 to 100 moles of ammonia and 0.01 to 100 moles of oxygen per mole of cyclohexanone. However, the stoichiometric ratios are cyrohexanone:ammonia:$O_2$=1:1:0.5 and since use of ammonia in large excess amounts increases costs for recovering unreacted ammonia from the reaction product gas and use of oxygen in large excess amounts may result in increased combustion of cyclohexanone or ammonia, the molar ratios of cyclohexanone:ammonia: $O_2$ are preferably 1 : (0.01 to 10) : (0.1 to 100) and more preferably 1 : (0.1 to 10) : (0.1 to 10). The mixed gas composed of cyclohexanone, ammonia and oxygen can be explosive so that it is usually preferred to dilute the mixed gas with an inert gas such as nitrogen, carbon dioxide or argon before it can be supplied to the reaction.

When urea is used as the ammonia generating source, water must be present in the reaction system in order to hydrolyze urea. However, in the reaction system water is generated by the oxidation reaction and as a result, water does not have to be added intentionally. The molar ratio of urea and ammonium salts in the starting materials supplied to the reaction may be set by calculating the amount of ammonia generated by the decomposition of them based on the aforementioned molar ratio of ammonia in the gas.

The existence of water in the reaction system can increase the selectivity of ε-caprolactam produced. In this case, the amount of water that exists in the reaction system is usually 0.001 to 1,000 moles, preferably 0.01 to 500 moles and more preferably 0.1 to 100 moles per mole of cyclohexanone as the substrate.

Similarly, the existence of carbon dioxide in the reaction system can increase the selectivity of ε-caprolactam. In this case, the amount of carbon dioxide that exists in the reaction system is usually, 0.001 to 1,000 moles, preferably 0.01 to 500 moles and more preferably 0.1 to 100 moles per mole of cyclohexanone as the substrate.

The reaction temperature is usually 150 to 400°C. The present invention is advantageous in that ε-caprolactam can be produced at a high selectivity at a relatively low temperature as compared with the reaction temperature of a conventional Beckmann rearrangement. In addition, according to the present invention, ε-caprolactam can be produced at a high selectivity in a single stage reaction region without using any conventionally known multi-stage reactor.

As the reaction device, a fixed bed reactor, a fluidized bed reactor and the like that are commonly used may be employed. In the reaction, carbon substances that are produced on the catalyst by side reactions will be deposited to decrease the catalyst activity. Therefore, it is preferred to use a fluidized bed reactor, with which the catalyst can be taken out or supplemented without difficulty. The catalyst whose activity has decreased can be subjected to any suitable method for removing the carbon substances, such as washing or burning, to thereby recover the activity.

The produced ε-caprolactam can be recovered from the reaction product gas that flows out of the reactor by any suitable means such as cooling or absorption with a solvent. Since the reaction product gas usually contains unreacted cyclohexanone and cyclohexanone oxime as a by-product, a solution in which ε-caprolactam is coexistent with the unreacted material and by-product is obtained upon cooling or absorption with a solvent. Accordingly, ε-caprolactam, cyclohexanone oxime and cyclohexanone are recovered by any suitable fractional recovery means such as distillation or crystallization. The recovered cyclohexanone is reused in the reaction and cyclohexanone oxime can be converted into ε-caprolactam by a known method.

As an alternative method, in accordance with the method described in J. Cat., 70, (1981) cited above, the reaction product gas that has flown out from the reactor can subsequently be brought in contact with a gas phase Beckmann rearrangement catalyst to convert cyclohexanone oxime contained into ε-caprolactam.

BRIEF DESCRIPTION OF THE DRAWINGS

Fig. 1 is a diagram schematically showing an automatic temperature programmed desorption analyzer (TP-5000, manufactured by Ookura Riken Co., Ltd.).

Fig. 2 is a diagram showing the structure of a reaction tube used in temperature programmed-desorption analysis using the temperature programmed desorption apparatus.

BEST MODE FOR CARRYING OUT THE INVENTION

Hereinafter, the present invention will be described in detail. However, the present invention is by no means limited thereto. The catalysts described below were prepared under sufficient stirring.

Preparation of Catalyst (1) ;

85.41 g of an aqueous 40 wt% tetrapropylammonium hydroxide (N-$(n-C_3H_7)_4$OH) solution containing sodium and potassium respectively in an amount under 10 ppm was dropped to 101.0 g of tetraethoxysilane (Si$(OC_2H_5)_4$) and a mixture was stirred for 45 minutes. A solution of 1.57 g of pentaisopropoxyniobium (Nb$(i-OC_3H_7)_5$) dissolved in 15.7 g of isopropanol was dropped to the mixture and the mixture was stirred for 75 minutes. Subsequently, while vigorously stirring, 210.5 g of deionized water was added. After 30 minute's stirring, the mixture was transferred to a Teflon vessel. The vessel was placed in an autoclave and the reaction was performed at 170°C for 96 hours with stirring at 90 rpm. The produced slurry was filtered and washed with water to obtain white solids, which were dried at 110°C for 1 day followed by calcined at 550°C for 8 hours in an air atmosphere to prepare a catalyst. As a result of powder X-ray diffraction, this was identified to be ZSM-5.

Preparation of Catalyst (2);

70.08 g of tetraethoxysilane and 75.61 g of an aqueous 40 wt% tetrabutylammonium hydroxide (N(n-$C_4H_9)_4$OH) solution were mixed and stirred for 1 hour. A solution of 2.59 g of pentaisopropoxyniobium dissolved in 26 g of isopropanol was dropped to a mixture, and the mixture was stirred for 1 hour. Subsequently, while vigorously stirring, 134.45 g of deionized water was added thereto, followed by 1 hour stirring. The reaction mixture was transparent (pH=11.11). The reaction mixture was refluxed at 80°C for 2 hours under nitrogen stream, and then transferred into a Teflon vessel. The vessel was placed in an autoclave and the temperature was elevated to 170°C and the reaction was performed for 72 hours while stirring at 88 rpm. The resultant slurry was filtered and washed with water to obtain white solids, which were dried at 120°C for 1 day and carcined at 550°C for 8 hours in an air atmosphere to prepare a catalyst. As a result of powder X-ray diffraction, this was identified to be ZSM-11.

Preparation of Catalyst (3);

100 g of tetraethoxysilane and 250 g of an aqueous 20 wt% tetrapropylammonium hydroxide solution were mixed and heated to 80°C to hydrolyze tetraethoxysilane and by-produced ethanol was distilled off. Then, the reaction mixture was transferred to a Teflon vessel, which was placed in an autoclave. The reaction was carried out at 180°C for 60 hours while stirring.

The resultant slurry was filtered and washed with water to obtain white solids, which were dried at 120°C for 1 day and calcined at 550°C for 4 hours in an air atmosphere. As a result of powder X-ray diffraction, this was identified to be ZSM-5. Hereinafter, this catalyst is also referred to as "Silicalite-1".

A solution of 0.22 g of pentaisoporopoxyniobium dissolved in 20 g of isopropanol was added to 4 g of Silicalite-1 obtained as above and further 25 g of deionized water was added. The mixture was stirred at 80°C for 5 hours. Under reduced pressure, water and isopropanol were distilled off and the obtained solids were dried at 120°C for 12 hours and calcined at 550°C for 8 hours in an air atmosphere to prepare a catalyst.

Preparation of Catalyst (4);

40 g of tetraethoxysilane, 33.2 g of an aqueous 40 wt% tetrapropylammonium hydroxide solution, and 11.65 g of an ethanolic 10 wt% penta-n-butoxytantalum (Ta(n-OC$_4$H$_9$)$_5$) solution were mixed. while ice-cooling the mixed solution, a mixture of 4.2g of an aqueous 40 wt% tetrapropylammonium hydroxide solution and 13 g of deionized water was slowly dropped thereto with stirring. After completion of the dropping, the temperature was elevated to 70°C and the mixture was stirred for 1 hour. Subsequently, while stirring, nitrogen gas was flown on the liquid surface to remove the by-produced alcohol. Then, 42 g of deionized water was added and the mixture was transferred to a Teflon vessel, which was placed in an autoclave. The reaction was carried out at 180°C for 120 hours without stirring. The resultant slurry was filtered and washed with water to obtain white solids, which were dried at 120°C for 1 day and calcined at 550°C for 4 hours in an air atmosphere to prepare a catalyst. As a result of powder X-ray diffraction, this was identified to be ZSM-5.

Preparation of Catalyst (5) ;

58.72 g of an aqueous 40 wt% tetrapropylammonium hydroxide solution was dropped to 68.74 g of tetraethoxysilane while vigorously stirring, and subsequently the resultant reaction mixture was stirred for 45 minutes. A mixture of a solution of 1.06 g of hafnium tetrachloride (HfCl$_4$) dissolved in 15 g of water and a solution of 1.23 g of oxalic acid dissolved in 15 g of water was added to the above reaction mixture and subsequently the resultant mixture was stirred for 75 minutes. 142.67 g of water was added to the mixture and the mixture was stirred for 1 hour. The pH of the solution then was 11.65.

This solution was transferred to a Teflon vessel, which was placed in an autoclave. The reaction was performed at 170°C for 96 hours with stirring at 90 rpm. The resultant slurry was filtered and washed with water to obtain white solids, which were dried at 110°C for 1 day and calcined at 550°C for 8 hours in an air atmosphere to prepare a catalyst. As a result of powder X-ray diffraction, this was identified to be ZSM-5.

Preparation of Catalyst (6);

85.41 g of an aqeuous 40 wt% tetrapropylammonium hydroxide solution was dropped to 101.0 g of tetraethoxysilane while vigorously stirring, and subsequently the resultant reaction mixture was stirred for 30 minutes. A solution of 1.36 g of tetrabutoxytitanium (Ti(n-O-C$_4$H$_9$)$_4$) dissolved in 10 g of isopropanol was dropped to the reaction mixture and subsequently the resultant mixture was stirred for 1 hour. While more vigorously stirring, 251.54 g of deionized water was added thereto and the mixture was stirred for additional 30 minutes. The pH of the solution then was 11.42.

This solution was transferred to a Teflon vessel, which was placed in an autoclave. The reaction was performed at 170°C for 96 hours with stirring at 90 rpm. The resultant slurry was filtered and washed with water to obtain white solids, which were dried at 110°C for 1 day and calcined at 550°C for 8 hours in an air atmosphere to prepare a catalyst. As a result of powder X-ray diffraction, this was identified to be ZSM-5.

Preparation of Catalyst (7);

58.72 g of an aqueous 40 wt% tetrapropylammonium hydroxide solution was dropped to 69.44 g of tetraethoxysilane while vigorously stirring, and subsequently the resultant reaction mixture was stirred for 45 minutes. A mixture of a solution of 0.61 g of ammonium paratungstate $((NH_4)_{10}W_{12}O_{41}\cdot5H_2O)$ dissolved in 15 g of water and a solution of 1.03 g of oxalic acid dissolved in 15 g of water was added to the reaction mixture, and subsequently the resultant mixture was stirred for 75 minutes. Then, 172.67 g of water was added and the mixture was stirred for 1 hour. The pH of the solution then was 11.85.
This solution was transferred to a Teflon vessel, which was placed in an autoclave. The reaction was performed at 170°C for 96 hours with stirring at 90 rpm. The resultant slurry was filtered and washed with water to obtain white solids, which were dried at 110°C for 1 day and calcined at 550°C for 8 hours in an air atmosphere to prepare a catalyst. As a result of powder X-ray diffraction, this was identified to be ZSM-5.

Preparation of Catalyst (8);

With reference to the method described in A. Tuel, et al., Chem. Commun. (1996) page 651, a catalyst of a hexagonal mesoporous silica structure containing zirconium was prepared as described below.
A solution of 7.24 g of hexadecylamine dissolved in 29.9 g of ethanol was added to a mixed solution containing 20.8 g of tetraethoxysilane and 1.64 g of tetraisopropoxyzirconium $(Zr(i-OC_3H_7)_4)$, and further 54 g of water was added. The resultant gel was stirred for 30 minutes and then allowed to stand for 12 hours for aging. The product was filtered, washed in water and then air-dried.
The obtained solids were dispersed in ethanol with a ratio of 100 ml of ethanol per g of solid and refluxed for 1 hour. After filtration, the obtained solids were washed with ethanol. The above procedure was repeated once again to sufficiently remove the contained amine. The obtained solids were dried at 80°C for 1 day and then calcined at 550°C for 5 hours to prepare a catalyst.

Preparation of Catalyst (9);

A solution obtained by dissolving 1.92 g of pentaisopropoxyniobum and 20.6 g of tetraethoxysilane in a mixed solution of 17.0 g of ethanol and 16.8 g of isopropanol was dropped to a mixed solution of 5.0 g of dodecylamine, 0.167 ml of concentrated hydrochloric acid solution, and 65.2 g of deionized water while stirring, and the mixture was stirred for 30 minutes. This was aged at room temperature for 18 hours and then filtered. The residue was air-dried. 450 ml of ethanol was added to 3 g of the obtained solids and the mixture was refluxed at 80°C for 1 hour in a nitrogen atmosphere. The solids obtained by filtration were washed with 300 ml of cold ethanol and air-dried. The reflux-washing-air drying treatment was performed once again and the solids were calcined at 550°C for 5 hours in an air atmosphere to prepare a catalyst. As a result of powder X-ray diffraction, a broad peak (interplanar spacing 3.68 nm) was observed in the vicinity of $2\theta=2.4°$, which suggested that this have a hexagonal mesoporous silica structure.

Preparation of Catalyst (10);

A solution of 0.61 g of tetraisopropoxytitanium $(Ti(i-OC_3H_7)_4)$ dissolved in 15 g of isopropanol was added to 3 g of Silicalite-1 obtained upon preparation of Catalyst (3) and the mixture was stirred to impregnate Silicalite-1 with the solution. Then, isopropanol was removed under reduced pressure and the obtained solids were dried at 120°C for 12 hours and further calcined at 550°C for 5 hours in an air atmosphere to prepare a catalyst.

Preparation of Catalyst (11);

0.84 g of urea and 0.96 g of tetraethoxysilane were added to a solution of 1.73 g of aluminum nitrate $(Al(NO_3)_3 9H_2O)$ dissolved in 5.03 g of methanol in order. 5.0 g of silica (CARIACT-50, particle size: 10 to 20 mesh, a product by Fuji Silicia Chemical Co.) was added to the resultant solution and the solution was subjected to pore-filling. Then, after drying under reduced pressure at 60°c, the obtained solids were calcined at 800°C for 4 hours in the air to prepare a catalyst.

Preparation of Catalyst (12);

A solution of 0.25 g of zirconium isopropoxide dissolved in 11.4 ml of isopropanol was dropped drop by drop to a solution of 18.9 g of tetraethoxysilane dissolved in 45.5 ml of isopropanol while vigorously stirring and the mixture was vigorously stirred for 30 minutes. Subsequently, while vigorously stirring the solution, 22.8 g of an aqueous 40 wt% tetrapropylammonium hydroxide solution was dropped thereto and subsequently the obtained reaction mixture was stirred for additional 60 minutes. While stirring more vigorously, 45.5 ml of deionized water was added thereto and the mixture was stirred for additional 60 minutes. The obtained solution was colorless and transparent.

Hydrothermal synthesis of this solution was practiced using a hydrothermal synthesis reactor KH-03SA, produced by Hiro Co., Ltd. As the microautoclave, a 200-cc microautoclave equipped with a Teflon vessel inside of the autoclave dedicated for this reactor was used. The rotary axis was rotated at a rotation speed of 15 rpm to stir the solution in the microautoclave. The temperature of oven was elevated to 183°C in 1 hour and this state was kept for 72 hours.

After the hydrothermal synthesis, the resultant slurry was filtered and washed with water to obtain white solids, which were dried at 120°C for 1 day, and then calcined at 550°C for 8 hours in an air atmosphere to prepare a catalyst.

Preparation of Catalyst (13);

55.56 g of tetraethoxysilane was dropped to 46.98 g of an aqueous 40 wt% tetrapropylammonium hydroxide solution with vigorous stirring, and subsequently the resultant reaction mixture was stirred for additional 45 minutes. A solution of 0.75 g of tetrabutoxytitanium ($Ti(n-O-C_4H_9)_4$) dissolved in 18 g of isopropanol was dropped to this reaction mixture, and subsequently the mixture was stirred for additional 1 hour. While more vigorously stirring, 114.37 g of deionized water was added thereto and the mixture was further stirred for 30 minutes. The solution was colorless and transparent. This solution was divided to two 200-cc microautoclaves and subjected to hydrothermal synthesis and then firing in the same manner as the catalyst (12) to prepare a catalyst.

Table 1 shows the metal composition, atomic ratio and structure of each of the catalysts obtained in the preparation of catalysts described above.

Table 1

| | Metal Composition | Atomic Ratio (Si*/M Molar Ratio)** | Structure |
|---|---|---|---|
| Catalyst 1 | Si-Nb | 120 | Crystalline composite oxide (ZSM-5) |
| Catalyst 2 | Si-Nb | 50 | Crystalline composite oxide (ZSM-11) |
| Catalyst 3 | Si-Nb | 120 | Silicalite-1 supporting Nb |
| Catalyst 4 | Si-Ta | 90 | Crystalline composite oxide (ZSM-5) |
| Catalyst 5 | Si-Hf | 100 | Crystalline composite oxide (ZSM-5) |
| Catalyst 6 | Si-Ti | 120 | Crystalline composite oxide (ZSM-5) |
| Catalyst 7 | Si-W | 120 | Crystalline composite oxide (ZSM-5) |
| Catalyst 8 | Si-Zr | 20 | Hexagonal mesoprous silica |
| Catalyst 9 | Si-Nb | 20 | Hexagonal mesoprous silica |
| Catalyst 10 | Si-Ti | 24 | Silicalite-1 supporting Ti |
| Catalyst 11 | Si-Al | 19 | Silica carrier supporting Al |
| Catalyst 12 | Si-Zr | 120 | Crystalline composite oxide (ZSM-5) |
| Catalyst 13 | Si-Ti | 120 | Crystalline composite oxide (ZSM-5) |

* M: Element selected from the elements belonging to the groups 3 to 12

** Charging ratio upon preparation of catalysts

Examples 1 to 13 and Comparative Examples 1 and 2

1 ml of a catalyst was packed in a quartz glass made tube type reaction tube having a length of 32 cm, an inner diameter of 6 mm and a length of 4.5 cm in the central portion and an inner diameter of 4 mm in the upper and lower portions, and preheated at a predetermined temperature for 1 hour. Then, a mixed gas of ammonia, cyclohexanone, and air was supplied under the conditions shown in Table 3 and subjected to reaction. The gas that flew from the reaction tube was ice-cooled to condense. The condensate was analyzed by gas chromatography. As the column, 5% PEG-HT/Uniport HP (60/80 mesh) 3 m-long glass made column was used and cyclopentanone was used as the internal standard.

In Example 13, after preheating the catalyst, a mixed gas of cyclohexanone and air was supplied under the conditions shown in Table 3 and an aqueous 20 wt% urea solution was simultaneously supplied at a rate of 0.35 ml/h. The analysis was performed in the same manner as described above.

Tables 2 and 3 show the particle size (mesh) of catalyst, reaction condition and reaction results used in each reaction described above. Conversion of cyclohexanone (CHN), selectivity of caprolactam (CL) and selectivity of cyclohexanone oxime (OXM) were obtained as follows.

CHN Conversion (%) = {(Mole number of supplied CHN -

mole number of recovered CHN)/(mole number of supplied

CHN)}×100

Selectivity of CL or OXM (%) = (Mole number of

produced CL or OXM/mole number of converted CHN)×100

Table 2

| | Catalyst | | | Preheating temperature (°C) |
|---|---|---|---|---|
| | Type | Particle size (mesh) | Charging amount (g) | |
| Example 1 | Catalyst (1) | 16-30 | 0.52 | 260 |
| Example 2 | Catalyst (2) | 16-30 | 0.52 | 350 |
| Example 3 | Catalyst (3) | 16-30 | 0.39 | 350 |
| Example 4 | Catalyst (4) | 10-20 | 0.37 | 260 |
| Example 5 | Catalyst (4) | 20-60 | 0.30 | 350 |
| Example 6 | Catalyst (5) | 10-20 | 0.50 | 260 |
| Example 7 | Catalyst (6) | 16-30 | 0.51 | 350 |
| Example 8 | Catalyst (8) | 16-30 | 0.52 | 260 |
| Example 9 | Catalyst (8) + Silicalite-1 | 20-60 | | 350 |
| Example 10 | Catalyst (9) | 10-20 | 0.26 | 350 |
| Example 11 | Catalyst (10) | 10-20 | 0.39 | 350 |
| Example 12 | Catalyst (12) | 16-30 | 0.41 | 350 |
| Example 13 | Catalyst (13) | 16-30 | 0.46 | 300 |
| Comparative Example 1 | Silicalite-1** | 10-20 | 0.39 | 260 |
| Comparative Example 2 | Catalyst (11) | 10-20 | 0.32 | 350 |

*: 0.5 ml of Catalyst (8) (particle size 20 to 60 mesh) was packed and 0.5 ml of Silicalite-1 (particle size 20 to 60 mesh) obtained in the preparation of catalyst (3) was packed subsequently at downstream thereof.

**: Silicalite-1 obtained in preparing Catalyst (3)

EP 1 142 871 A1

Table 3

| | Catalyst Composition (Si/M Molar ratio) | NH₃ Desorption Amount mmol-NH₃ /g-cat | Gas composition (0°C 1atm conversion ) CHN/NH₃/O₂/N₂ | Temperature °C | Time (hr) | CHN Conversion (%) | CL Selectivity (%) | OXM Selectivity (%) |
|---|---|---|---|---|---|---|---|---|
| Example 1 | Nb-Si MFL(120) | 0.02 | 1.2/0.8/1.5/14 | 250 | 3.5-4.5 | 9-18 | 14-32 | 7-16 |
| Example 2 | Nb-Si MEL(50) | <0.03 | 3.1/1.1/1.7/6.4 | 250 | 2.0-3.0 | 6 | 13 | 14 |
| Example 3 | Nb/Silicaite-1(120) | <0.08 | 1.6/1.2/1.9/7.1 | 250 | 2.0-3.0 | 36-43 | 11-14 | 2 |
| Example 4 | Ta-Si MFI(90) | <0.02 | 1.3/1.0/1.8/6.7 | 250 | 1.0-2.0 | 13 | 13 | 8 |
| | | | | 250 | 2.0-3.0 | 19 | 10 | 11 |
| Example 5 | Ta-Si MFI(90) | <0.02 | 1.3/0.1/1.8/6.7 | 250 | 1.0-2.0 | 13 | 14 | 7 |
| | | | | 250 | 2.0-3.0 | 12 | 20 | 8 |
| Example 6 | Hf-Si MFI(100) | 0.11 | 1.3/1.0/1.8/6.7 | 250 | 1.0-2.0 | 12 | 13 | 18 |
| | | | | 250 | 2.0-3.0 | 12 | 9 | 42 |
| Example 7 | Ti-Si MFI(120) | 0.01 | 1.3/1.0/1.8/6.7 | 250 | 1.0-2.0 | 10 | 27 | 22 |
| | | | | 250 | 2.0-3.0 | 24 | 12 | 20 |
| Example 8 | W-Si MFI(120) | 0.003 | 1.3/0.9/0.47/5.6 | 260 | 1.0-2.0 | 5 | 16 | 1 |
| | | | | 260 | 2.0-3.0 | 4 | 25 | 4 |
| Example 9 | Zr-SiHMS+Silicalite-1 | <0.21 | 1.3/0.1/1.8/12.2 | 250 | 1.0-2.0 | 4 | 46 | 34 |
| | | | | 250 | 2.0-3.0 | 9 | 13 | 11 |
| Example 10 | Nb-Si HMS(20) | <0.27 | 1.3/1.0/1.8/6.7 | 260 | 2.5-3.5 | 43 | 8 | 18 |
| Example 11 | Ti/Silicalite(24) | <0.01 | 1.3/1.0/1.8/6.7 | 260 | 1.0-2.0 | 22 | 6 | 2 |
| | | | | 260 | 2.0-3.0 | 32 | 3 | 3 |
| Example 12 | Zr-Si MFI(120) | 0.25 | 1.3/0.1/1.8/6.7 | 250 | 1.0-2.0 | 5 | 9 | 9 |
| | | | | 250 | 2.0-3.0 | 8 | 7 | 22 |
| Example 13 | Si-Ti MFI(120) | 0.01 | 1.3/0/1.4/14.2 | 300 | 1.0-2.0 | 3 | 69 | 0 |
| Comparative Example 1 | Silicalite-1 | <0.03 | 1.3/1.0/1.8/6.7 | 250 | 2.0-3.0 | 25 | 5 | 3 |
| Comparative Example 2 | SiO₂-Al₂O₃(19) | <0.22 | 1.3/1.0/1.8/6.7 | 257 | 2.5-3.5 | 35 | 2 | 7 |

EP 1 142 871 A1

INDUSTRIAL APPLICABILITY

By using specified oxide catalysts of the present invention, ε-caprolactam can be produced in one step and at a high selectivity, so that they are highly useful in industry.

**Claims**

1. A method for producing ε-caprolactam comprising allowing cyclohexanone to react with a nitrogen source and oxygen in the presence of an oxide catalyst containing one or more elements M(s) selected from elements belonging to the groups 3 to 12 in the periodic table.

2. A method for producing ε-caprolactam according to claim 1, wherein the oxide catalyst further comprises silicon.

3. A method for producing ε-caprolactam according to claim 1 or 2, wherein the reaction is carried out in a gas phase.

4. A method for producing ε-caprolactam according to any one of claims 1 to 3, wherein the nitrogen source is ammonia or an ammonia generating source.

5. A method for producing ε-caprolactam according to any one of claims 1 to 4, wherein the oxide catalyst has an ammonia desorption amount of 0.5 mmol-$NH_3$/g-cat or less in temperature programmed desorption analysis of ammonia.

6. A method for producing ε-caprolactam according to claim 5, wherein the oxide catalyst has an ammonia desorption amount of 0.3 mmol-$NH_3$/g-cat or less in temperature programmed desorption analysis of ammonia.

7. A method for producing ε-caprolactam according to any one of claims 1 to 6, wherein the oxide catalyst has an aluminum content of 1 wt% or less.

8. A method for producing ε-caprolactam according to claim 7, wherein the oxide catalyst has an aluminum content of 0.5 wt% or less.

9. A method for producing ε-caprolactam according to any one of claims 1 to 8, wherein the element M comprises at least one element selected from elements belonging to the groups 4 to 8 in the periodic table.

10. A method for producing ε-caprolactam according to claim 9, wherein the element M comprises at least one element selected from Ti, Nb, Ta, Hf, Zr and W.

11. A method for producing ε-caprolactam according to any one of claims 2 to 10, wherein the oxide catalyst has an atomic ratio of silicon to element M (Si/M) of 20 to 190.

12. A method for producing ε-caprolactam according to claim 11, wherein the oxide catalyst has an atomic ratio of silicon to element M (Si/M) of 50 to 150.

13. A method for producing ε-caprolactam according to any one of claims 1 to 12, wherein the oxide catalyst is a crystalline oxide.

14. A method for producing ε-caprolactam according to any one of claims 1 to 13, wherein the oxide catalyst is a composite oxide.

15. A method for producing ε-caprolactam according to claim 14, wherein the oxide catalyst is zeolite.

16. A method for producing ε-caprolactam according to any one of claims 1 to 15, wherein the reaction is performed at 150 to 320°C.

17. A method for producing ε-caprolactam according to any one of claims 1 to 16, wherein the gas phase is a gas containing 0.01 to 10 moles of the nitrogen source and 0.1 to 100 moles of oxygen per mole of cyclohexanone.

18. A method for producing ε-caprolactam according to any one of claims 1 to 17, wherein the reaction is performed in a single reaction region.

19. A method for producing ε-caprolactam according to any one of claims 1 to 18, wherein a reaction product gas containing ε-caprolactam produced by the reaction is cooled or absorbed with a solvent to obtain a solution containing ε-caprolactam, from which solution ε-caprolactam is recovered.

20. A method for producing ε-caprolactam according to any one of claims 1 to 19, wherein a reaction product gas containing ε-caprolactam produced by the reaction and by-produced cyclohexanone oxime is brought into contact with a gas phase Beckmann rearrangement catalyst to convert at least a portion of cyclohexanone oxime contained in the gas into ε-caprolactam.

21. An oxide catalyst for producing ε-caprolactam having the following characteristics 1) to 3):

1) containing one or more elements M(s) selected from elements belonging to the groups 3 to 12 in the periodic table;
2) having an ammonia desorption amount of 0.5 mmol-$NH_3$/g-cat or less in temperature programmed desorption analysis of ammonia; and
3) having an aluminum content of 1 wt% or less.

22. An oxide catalyst for producing ε-caprolactam according to claim 21, further comprising silicon.

23. An oxide catalyst according to claim 21 or 22, wherein the ammonia desorption amount is 0.3 mmol-$NH_3$/g-cat or less.

24. An oxide catalyst according to any one of claims 21 to 23, wherein the oxide catalyst has an aluminum content of 0.5 wt% or less.

25. An oxide catalyst according to any one of claims 21 to 24, wherein the element M comprises at least one element selected from elements belonging to the groups 4 to 8 in the periodic table.

26. An oxide catalyst according to claim 25, wherein the element M comprises at least one element selected from Ti, Nb, Ta, Hf, zr and W.

27. An oxide catalyst according to any one of claims 21 to 26, wherein the oxide catalyst has an atomic ratio of silicon to element M (Si/M) of 20 to 190.

28. An oxide catalyst according to claim 27, wherein the oxide catalyst has an atomic ratio of silicon to element M (Si/M) of 50 to 150.

29. An oxide catalyst according to any one of claims 21 to 28, wherein the oxide catalyst is a crystalline oxide.

30. An oxide catalyst according to any one of claims 21 to 29, wherein the oxide catalyst is a composite oxide.

31. An oxide catalyst according to claim 30, wherein the oxide catalyst is zeolite.

# FIG. 1

FIG. 2

<div style="text-align:center">

**INTERNATIONAL SEARCH REPORT**

</div>

| International application No. |
| --- |
| PCT/JP99/07301 |

| A. CLASSIFICATION OF SUBJECT MATTER |
| --- |
| Int.Cl$^7$   C07D201/04, B01J29/40 // C07B61/00 |
| According to International Patent Classification (IPC) or to both national classification and IPC |

| B.   FIELDS SEARCHED |
| --- |
| Minimum documentation searched (classification system followed by classification symbols)<br>Int.Cl$^7$   C07D201/04, B01J29/40 // C07B61/00 |
| Documentation searched other than minimum documentation to the extent that such documents are included in the fields searched |
| Electronic data base consulted during the international search (name of data base and, where practicable, search terms used)<br>CA, REGISTRY (STN) |

| C. DOCUMENTS CONSIDERED TO BE RELEVANT | | |
| --- | --- | --- |
| Category* | Citation of document, with indication, where appropriate, of the relevant passages | Relevant to claim No. |
| X | PRASAD R. et al., "Ammoximation of cyclohexanone by nitric oxide and ammonia : A one-step process for synthesis of poly-caprolactam", J.Catal., (1996), 161(1), p.373-6 | 1-31 |
| X | THANGARAJ A. et al., "Catalytic properties of crystalline titanium silicalites", J.Catal., (1991), 131(2), p.394-400 | 1-31 |
| X<br>Y | USHIKUBO T. et al., "Vapor phase Beckmann rearrangement over silica-supported tantalum oxide catalyst", J.Catal., (1994), 148(1), p.138-48 | 21-31<br>1-20 |
| X<br>Y | KATADA N. et al., "Vapor-phase Beckmann rearrangement over silica monolayers prepared by chemical vapor deposition", Appl.Catal., A, (1995), 124(1), p.1-7 | 21-31<br>1-20 |
| X | WO, 97/3956, A1 (Mitsubishi Chemical Corporation), 06 February, 1997 (06.02.97), Full text   & US, 5900482, A | 21-31 |
| A | JP, 47-20183, A (Mitsui Toatsu Chemicals Inc.), 27 September, 1972 (27.09.72)   (Family: none) | 1-31 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>24 March, 2000 (24.03.00) | Date of mailing of the international search report<br>04 April, 2000 (04.04.00) |
| --- | --- |
| Name and mailing address of the ISA/<br>Japanese Patent Office | Authorized officer |
| Facsimile No. | Telephone No. |

Form PCT/ISA/210 (second sheet) (July 1992)

## INTERNATIONAL SEARCH REPORT

| International application No. |
| --- |
| PCT/JP99/07301 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | JP, 49-9472, B (Asahi Chemical Industry Co., Ltd.), 05 March, 1974 (05.03.74) (Family: none) | 1-31 |

Form PCT/ISA/210 (continuation of second sheet) (July 1992)